# EUROPEAN PATENT SPECIFICATION

(11) **EP 3 691 718 B1**
(45) Date of publication and mention of the grant of the patent: **07.08.2024**
(21) Application number: 18812326.9
(22) Date of filing: 01.10.2018
(51) Int. Cl.: A61M 5/172, A61M 39/24, A61B 5/145, A61M 5/36, A61B 5/00, A61M 5/14, A61M 5/168, A61M 39/10, A61M 5/158

(54) **DEVICE FOR REGULATING THE CONCENTRATION OF GLUCOSE IN THE BLOOD OF A PERSON**
VORRICHTUNG ZUR REGELUNG DER KONZENTRATION VON GLUCOSE IM BLUT EINER PERSON
DISPOSITIF POUR RÉGULER LA CONCENTRATION EN GLUCOSE DANS LE SANG D'UNE PERSONNE

(30) Priority: 03.10.2017 NL 2019661
(43) Date of publication of application: 12.08.2020
(73) Proprietor: Inreda Diabetic B.V., 7472 DD Goor (NL)
(72) Inventor: KOOPS, Robin, 7471 LW Goor (NL); WESTEN, Gijs Benno, 7627 PT Bornerbroek (NL); BOULAY, Mickael Gilbert Robert, 7411 KP Deventer (NL)
(86) International application number: PCT/NL2018/050648
(87) International publication number: WO 2019/083354

(56) References cited:
- EP-A1- 0 621 791
- WO-A1-2006/001759
- WO-A1-2014/204529
- WO-A1-99/29356
- WO-A2-2007/049961
- US-A1- 2008 065 006
- US-A1- 2008 195 060

## Description

The invention relates to a device for regulating the concentration of glucose in the blood of a person, comprising:
- pump means for selectively supplying at least one substance which affects the concentration of glucose in the blood of the person;
- injecting means for injecting the substance into the body of the person, such as a cannula or catheter, and
- transport means, such as a conduit, tube, hose or the like, which connect the pump means for medium throughflow to the injecting means for the purpose of transporting the substance from the pump means to the injecting means.

Such a device is per se known. Examples of such a device are an insulin pump or an artificial pancreas.

It is an object of the invention to improve the per se known device.

This object is achieved with a device of the type stated in the preamble, which is configured according to the invention to flush out the transport means with the substance, and wherein the device comprises detection means for detecting whether or not the device is in medium throughflow contact with the internal part of the body of the person via the transport means and the injecting means, wherein the device is configured to flush out the transport means only when the detection means detect that there is no medium throughflow contact with the internal part of the body of the person.

The flushing out of the transport means can for instance be desired prior to use of a new set of transport means. At that moment the transport means may be filled with air, which air is preferably expelled from the transport means before they are taken into use. It can alternatively or additionally be desirable to flush out transport means which have already been taken into use, for instance periodically after passage of a determined period of use, or for instance when a container for containing the substance is empty and a new, filled container is connected, or when a blockage is detected.

It is advantageous in both cases for the transport means to be flushed out only when it is detected that there is no contact with the internal part of the body of the person, since, in the case of a new set of transport means, the air would otherwise be pumped into the body and, in the case of a set of transport means already filled with substance, too much substance could be pumped into the body of the person. The detection means according to the invention are provided for this purpose.

It is noted that medium throughflow contact with the internal part of the body can at least be understood to mean that the substance can be injected into the body of the person when this contact exists, and that no substance can be injected into the body of the person when this contact does not exist. The contact will exist when the transport means are connected to both the pump means and the injecting means and when the injecting means are arranged in the body of the person, for instance subcutaneously.

Where reference is made in this application to connected, this can be understood to mean that there is a medium throughflow connection.

It is further noted that detecting whether or not the device is in medium throughflow contact with the internal part of the body of the person via the transport means and the injecting means can at least be understood to mean that the detection means can be configured to be able to detect that there is no medium throughflow contact with the internal part of the body of the person, without being able to positively ascertain that there is medium throughflow contact.

The detection means according to the invention can take any desired form.

The detection means can alternatively be referred to as detector.

In an embodiment of the device according to the invention the detection means are configured to detect whether or not the transport means are connected for medium throughflow to the injecting means, wherein the detection means are configured to determine that there is no contact with the internal part of the body of the person when it is detected that the transport means are not connected for medium throughflow to the injecting means.

If the transport means are not connected for medium throughflow to the injecting means, then the device is not in medium throughflow contact with the internal part of the body, regardless of whether or not the injecting means are arranged in the body of the person. This is therefore a simple and/or effective way of determining that there is no contact with the internal part of the body of the person.

Another advantage of this embodiment can be that the flushing out can take place only when the transport means are not connected for medium throughflow to the injecting means, whereby air or substance present in the transport means will not be pumped through the injecting means when they are flushed out.

Detection of whether or not the transport means are connected for medium throughflow to the injecting means can take place in any suitable manner.

Detection takes place particularly by the device itself and not by a user input, since this latter can be susceptible to errors.

In an embodiment of the device according to the invention this takes place in that the injecting means and the transport means are each provided with electrical or light-conducting contact means, which electrical or light-conducting contact means of the injecting means and the transport means are in mutual contact when the transport means are connected to the injecting means and thus close an electrical circuit or a light circuit, wherein the detection means are configured to determine that there is no contact with the internal part of the body of the person when it is detected that no electrical circuit or light circuit is closed.

The electrically conductive contact means can for instance comprise two electrically conductive wires, wherein the one wire is arranged in or on the transport means and debouches in a first coupling element of the transport means, for instance a coupling end, which will be connected to the injecting means, and wherein the other wire is arranged in or on the injecting means and debouches in a second coupling element of the injecting means, for instance a coupling end, which will be connected to the transport means. When the transport means will be connected by means of the first coupling element to the second coupling element of the injecting means, the electrically conductive wires will be in mutual contact and thus close the electrical circuit.

The light-conducting contact means can for instance comprise two light-conducting optical fibres, wherein the one fibre is arranged in or on the transport means and debouches in a first coupling element of the transport means, for instance a coupling end, which will be connected to the injecting means, and wherein the other fibre is arranged in or on the injecting means and debouches in a second coupling element of the injecting means, for instance a coupling end, which will be connected to the transport means. When the transport means will be connected by means of the first coupling element to the second coupling element of the injecting means, the light-conducting fibres will be in mutual contact and thus close the light circuit.

In yet another embodiment of the device according to the invention the device further comprises a sensor arranged on or in the body of the person, wherein one of the pump means and the sensor is configured to generate a signal and wherein the other of the pump means and the sensor is configured to receive a generated signal, wherein the detection means are configured to determine that there is no contact with the internal part of the body when a generated signal is not received.

The signal which is generated by the pump means can be transported via substance in the transport means and the injecting means and via the body, for instance via bodily fluids, and be received by the sensor, and vice versa when the sensor generates the signal and the pump means can receive this signal. If there is no contact with the internal part of the body, the signal will not be conveyed to respectively the sensor and the pump means and will not be received thereby, whereby it is detected that there is no contact with the internal part of the body of the person.

This embodiment is particularly suitable when substance is already present in the transport means, i.e. when transport means which have already been taken into use are flushed out.

The signal can be any suitable type of signal which can be conveyed through the substance and/or the body, for instance an ultrasonic signal or a light signal.

The pump means or the sensor can comprise transmitting means, for instance a transmitter, for transmitting or generating the signal. The other of the pump means and the sensor can comprise receiving means, for instance a receiver, for receiving the signal.

The sensor can be any suitable sensor for generating or receiving a signal. The sensor can for instance form part of a glucose sensor which can be provided when the device according to the invention is an artificial pancreas.

The sensor can for instance comprise a transmitter for transmitting to the pump means whether or not the signal has been received or for transmitting to the pump means when the signal has been received. In such an embodiment the sensor can comprise a receiver for receiving the signal and a transmitter for notifying the pump means whether or not the signal has been received or for notifying the pump means when the signal has been received.

In yet another embodiment of the device according to the invention the device is configured to determine the quantity of substance with which the transport means have to be flushed out in order to flush them out substantially wholly, and wherein the pump means are configured to pump the determined quantity of substance through the transport means.

An advantage of this embodiment is that it is prevents too little substance being used for flushing out the transport means, which can result in air or old substance remaining present in the transport means, but also prevents too much being used, which results in unnecessary loss of substance, in particular in that no more substance is pumped through the transport means than is necessary for flushing it out substantially wholly.

The determined quantity of substance can particularly be substantially equal to the internal volume of the transport means.

The device is particularly configured to determine the quantity of substance independently, and not be based on user input, since errors may be made during input.

In a practical embodiment the transport means are provided with indicating means for indicating a length and/or internal cross-sectional dimension and/or internal volume thereof, wherein the device is configured to determine the quantity of substance on the basis of the indicating means.

The length and/or internal cross-sectional dimension and/or internal volume of the transport means can be indicative of the quantity of substance needed to substantially wholly flush the transport means. On the basis of the indicating means the device knows the length and or internal cross-sectional dimension and/or internal volume, and can determine said quantity of substance.

The transport means can optionally have a fixed, standard internal cross-sectional dimension but have a variable length. The user can for instance choose from transport means with a length of 60, 80 or 100 cm. Because the internal cross-sectional dimension is fixed, the length thereof suffices to be able to determine the internal volume of the transport means, whereby the indicating means need only indicate the length.

If the transport means can have a variable cross-sectional dimension it is advantageous for the indicating means to indicate both the length and the internal cross-sectional dimension whereby the internal volume of the transport means can be determined, or for the indicating means to indicate the internal volume.

The indicating means can take any suitable form.

The transport means can for instance be provided with such indicating means on a third coupling element for coupling to the pump means. The indicating means can for instance comprise at least one protrusion or the like. The shape of the protrusion and/or the dimensions of the protrusion and/or the number of protrusions can be indicative of a length and/or internal cross-sectional dimension and/or internal volume of the transport means.

According to yet another example the indicating means can take an electronic form and comprise for instance a data chip. The data chip can comprise data about the transport means, particularly about for instance a length and/or internal cross-sectional dimension and/or internal volume thereof, and on the basis of these data the device can be configured to determine the quantity of substance. These electronic indicating means can alternatively also be referred to as data means.

In yet another embodiment of the device according to the invention the device comprises second detection means for detecting the possible presence of air in the transport means, and wherein the pump means are configured to flush out the transport means with the substance until the second detection means detect substantially no air in the transport means.

An advantage of this embodiment is that the device is configured to stop flushing out as soon as it is detected that substantially no air is present in the transport means, which is a sign that the transport means have been substantially wholly flushed out. This prevents too little substance being used for the flushing out, which results in air still being present therein, but also prevents too much being used, which results in substance being wasted.

Such an embodiment is particularly advantageous for a new set of transport means, which can be filled with air prior to a first use.

Detecting the possible presence of air in the transport means can take place in any suitable manner.

The second detection means can for instance be configured to convey a signal via substance present in the transport means during use and to receive this signal at an end of the transport means remote from the pump, wherein the second detection means are configured to determine that substantially no air is present in the transport means when the signal is received and is therefore conveyed to the end of the transport means.

When the transport means are completely filled with substance and no more air is present therein, the signal will be conveyed to the end of the transport means and will be received there, which is a sign that the flushing out can be stopped. If air is still present in the transport means the signal will not be conveyed due to the air, therefore will not be transported to the end of the transport means and will thus not be received at the end of the transport means. In this case the flushing out will continue until the signal is received.

The signal can be any suitable signal which can be transported through the substance, for instance an ultrasonic or light signal.

In yet another embodiment of the device according to the invention the device comprises third detection means for detecting whether or not the transport means are connected to the pump means, wherein the pump means are configured to pump substance only when it is detected that the transport means are connected to the pump means.

An advantage of this embodiment can be that the flushing out of the transport means will take place only when the transport means are connected to the pump means.

Another advantage hereof can be that leakage of substance can be prevented in that the pump means will stop pumping substance when or as soon as it is detected that there is no connection between the transport means and the pump means.

The detection of whether or not the transport means are connected for medium throughflow to the pump means can take place in any suitable manner.

The detection takes place particularly by the device itself and not by a user input, since this latter can be susceptible to errors.

In an embodiment of the device according to the invention this takes place in that the pump means and the transport means are each provided with second electrical or light-conducting contact means, which second electrical or light-conducting contact means of the pump means and the transport means are in mutual contact when the transport means are connected to the pump means and thus close a second electrical circuit or light circuit, wherein the third detection means are configured to detect that the transport means are connected for medium throughflow to the pump means when the second electrical circuit or light circuit is closed.

The second electrically conductive contact means can for instance comprise two second electrically conductive wires, wherein the one second wire is arranged in or on the transport means and debouches in a fourth coupling element of the transport means, for instance a coupling end, which will be connected to the pump means, and wherein the other second wire is arranged in or on the pump means and debouches in a fifth coupling element of the pump means, for instance a coupling end, which will be connected to the transport means. When the transport means will be connected by means of the fourth coupling element to the fifth coupling element of the injecting means, the second electrically conductive wires will be in mutual contact and thus close the electrical circuit.

The second light-conducting contact means can for instance comprise two second light-conducting optical fibres, wherein the one second fibre is arranged in or on the transport means and debouches in a first coupling element of the transport means, for instance a coupling end, which will be connected to the injecting means, and wherein the other second fibre is arranged in or on the injecting means and debouches in a second coupling element of the injecting means, for instance a coupling end, which will be connected to the transport means. When the transport means will be connected by means of the first coupling element to the second coupling element of the injecting means, the second light-conducting fibres will be in mutual contact and thus close the light circuit.

In yet another embodiment of the device according to the invention the device further comprises a second sensor arranged on or in the body of the person, wherein one of the pump means and the second sensor is configured to generate a signal and wherein the other of the pump means and the second sensor is configured to receive a generated signal, wherein the third detection means are configured to determine that the transport means are connected for medium throughflow to the pump means when the generated signal is received.

The signal which is generated by the pump means can be transported via substance in the transport means and the injecting means and via the body, for instance via a bodily fluid, and be received by the second sensor, and vice versa when the second sensor generates the signal and the pump means can receive this signal. If the transport means are not connected for medium throughflow to the pump means, the signal will not be conveyed to respectively the second sensor and the pump means and will not be received thereby.

This embodiment is particularly suitable when substance is already present in the transport means, i.e. when transport means which have already been taken into use are flushed out.

This embodiment can be particularly suitable for being able to detect during use that the connection between the transport means and the pump means has been released, for instance by the connection therebetween coming loose, and for preventing leakage of substance, in that the pump means will in that case stop pumping substance.

The signal can be any suitable type of signal which can be conveyed through the substance and/or the body, for instance an ultrasonic signal or a light signal.

The pump means or the second sensor can comprise transmitting means for transmitting or generating the signal. The other of the pump means and the second sensor can comprise receiving means for receiving the signal.

The second sensor can be any suitable sensor for generating or receiving a signal. The second sensor can for instance form part of a glucose sensor which can be provided when the device according to the invention is an artificial pancreas.

The second sensor can for instance comprise a second transmitter for transmitting to the pump means whether or not the signal has been received or for transmitting to the pump means when the signal has been received. In such an embodiment the second sensor can comprise a second receiver for receiving the signal and a second transmitter for notifying the pump means whether or not the signal has been received or for notifying the pump means when the signal has been received.

If desired, the sensor and the second sensor can be formed by one sensor.

The invention also relates to the use of a device according to one or more embodiments or with one or more of the features as described in this text.

The device according to the invention can further comprise a processor for controlling the device, for instance on the basis of the detection means and/or the second detection means and/or the third detection means.

The device according to the invention can further comprise at least one container for containing the substance. If desired, this container can be connected releasably to the device, particularly to the pump means, so that an empty container can be exchanged for a filled container. If desired, a plurality of containers can be provided, these containing if desired the same or different substances. One container with glucagon and one container with insulin can for instance be provided.

The device can also comprise two transport means and two injecting means, one for transporting and injecting a first substance, for instance insulin, and one for transporting and injecting a second, other substance, for instance glucagon. For the sake of convenience these are each referred to as an infusion set. Separate detection means and/or second detection means and/or third detection means as described above and/or having one or more of the features as described above can if desired be provided per infusion set. If desired, the generated signals can differ per infusion set and/or per substance, so that the device is configured to be able to determine whether or not a signal is received, and in which infusion set.

It is noted that substance can be pumped through the transport means both in injection and in flushing out. In the flushing out this takes place at least with the object of flushing out the transport means, and at least part of this substance can be injected into the body later, after the device has been connected to the internal part of the body of the person. In the injecting this takes place at least with the object of injecting the substance into the body of the person.

The invention likewise relates to a method for regulating the concentration of glucose in the blood of a person, comprising the use of a device comprising:
- pump means for selectively supplying at least one substance which affects the concentration of glucose in the blood of the person;
- injecting means for injecting the substance into the body of the person, such as a cannula or catheter, and
- transport means, such as a conduit, tube, hose or the like, which connect the pump means for medium throughflow to the injecting means for the purpose of transporting the substance from the pump means to the injecting means;
wherein the method comprises the steps of:
- flushing out the transport means with the substance, and
- detecting whether or not the device is in medium throughflow contact with the internal part of the body of the person via the transport means and the injecting means, wherein flushing out of the transport means is performed only when the detection means detect that there is no medium throughflow contact with the internal part of the body of the person.

The method, optionally according to one or more of the embodiments stated below, can use any device with any feature as described in this text, in any combination.

The method, optionally according to one or more of the embodiments stated below, can perform said steps in any suitable order.

The advantages of such a method are described above on the basis of the device according to the invention.

In an embodiment of the method according to the invention the method comprises the steps of:
- detecting whether or not the transport means are connected for medium throughflow to the injecting means,
- determining that there is no contact with the internal part of the body of the person when it is detected that the transport means are not connected for medium throughflow to the injecting means.

In another embodiment of the method according to the invention the method comprises the steps of:
- generating a signal and transporting this generated signal via substance in the transport means and/or the injecting means and/or via the body, for instance via a bodily fluid;
- optionally receiving the generated and transported signal, and
- determining that there is no contact with the internal part of the body of the person when a generated signal is not received.

In another embodiment of the method according to the invention the method comprises the steps of:
- determining the quantity of substance with which the transport means have to be flushed out in order to flush them out substantially wholly; and
- pumping the determined quantity of substance through the transport means.

In another embodiment of the method according to the invention the method comprises the steps of:
- detecting the possible presence of air in the transport means, and
- flushing out the transport means with the substance until it is detected that substantially no air is present in the transport means.

In another embodiment of the method according to the invention the method comprises the steps of:
- conveying a signal via substance present in the transport means;
- optionally receiving this signal at an end of the transport means remote from the pump, and
- determining that substantially no air is present in the transport means when the signal is received and is therefore conveyed to the end of the transport means.

In another embodiment of the method according to the invention the method comprises the steps of:
- detecting whether or not the transport means are connected to the pump means, and
- pumping substance only when it is detected that the transport means are connected to the pump means.

In another embodiment of the method according to the invention the method comprises the steps of:
- generating a signal and transporting this generated signal via substance in the transport means and/or the injecting means and/or via the body, for instance via a bodily fluid;
- optionally receiving the generated and transported signal, and
- determining that the transport means are connected for medium throughflow to the pump means when the generated signal is received.

The invention can also relate to a device for regulating the concentration of glucose in the blood of a person, comprising:
- pump means for selectively supplying at least one substance which affects the concentration of glucose in the blood of the person;
- injecting means for injecting the substance into the body of the person, such as a cannula or catheter,
- transport means, such as a conduit, tube, hose or the like, which connect the pump means for medium throughflow to the injecting means for the purpose of transporting the substance from the pump means to the injecting means, and
- third detection means for detecting whether or not the transport means are connected to the pump means, wherein the pump means are configured to pump substance only when it is detected that the transport means are connected to the pump means.

The third detection means can be embodied as described above and comprise one or more of the above described features, alone or in any suitable combination. The advantages of such third detection means, such as for instance the prevention of leakage of substance, are elucidated above.

The invention can also relate to a device for regulating the concentration of glucose in the blood of a person, comprising:
- pump means for selectively supplying at least one substance which affects the concentration of glucose in the blood of the person;
- injecting means for injecting the substance into the body of the person, such as a cannula or catheter,
- transport means, such as a conduit, tube, hose or the like, which connect the pump means for medium throughflow to the injecting means for the purpose of transporting the substance from the pump means to the injecting means;
- detection means for detecting whether or not the device is in medium throughflow contact with the internal part of the body of the person via the transport means and the injecting means, wherein the device is configured to inject a substance into the body of the person only when the detection means detect that there is medium throughflow contact with the internal part of the body of the person.

According to this invention, the detection means for detecting whether or not the device is in medium throughflow contact with the internal part of the body of the person via the transport means and the injecting means are used for controlling the normal dispensing of substance, i.e. for injecting the substance into the body of the person. The device can be configured here to use a positive detection, wherein it is determined that there is contact with the internal part of the body, to determine that the substance can be injected into the body. As soon as or if it is detected that there is no medium throughflow contact with the internal part of the body of the person, the injecting can be stopped so that no substance will leak and/or be lost.

In this invention the detection means can be embodied as described above and comprise one or more of the above described features, alone or in any suitable combination.

In this invention the detection means can in particular comprise the sensor arranged on the person, wherein one of the pump means and the sensor is configured to generate a signal and wherein the other of the pump means and the sensor is configured to receive a generated signal, wherein the detection means are configured to determine that there is contact with the internal part of the body of the person when a generated signal is received.

The invention can also relate to a device for regulating the concentration of glucose in the blood of a person, comprising:
- pump means for selectively supplying at least one substance which affects the concentration of glucose in the blood of the person;
- injecting means for injecting the substance into the body of the person, such as a cannula or catheter,
- transport means, such as a conduit, tube, hose or the like, which connect the pump means for medium throughflow to the injecting means for the purpose of transporting the substance from the pump means to the injecting means,
wherein the device is configured to determine the quantity of substance with which the transport means have to be flushed out in order to flush them out substantially wholly, and wherein the pump means are configured to pump the determined quantity of substance through the transport means.

Determining the quantity of substance can take place as elucidated above according to any of the described embodiments or with any feature as described above.

In this invention it is possible for the person him/herself to input that the device is not connected to the internal part of his/her body, after which the device is configured to flush out the transport means with the determined quantity of substance on the basis of this input.

It is noted that where reference is made in this text to first, second, third, fourth or fifth, this is used only to indicate and distinguish different elements, but these need not necessarily all be provided.

The third detection means can thus for instance be the only detection means.

The invention will be further elucidated with reference to figures, wherein:
Figure 1 shows schematically a prior art device;
Figure 2 shows schematically a part of the device according to a first embodiment of the invention;
Figure 3 shows schematically a part of the device according to a second embodiment of the invention, and
Figure 4 shows a block diagram of a method according to the invention.

In the figures the same components are designated with the same reference numerals increased by 100.

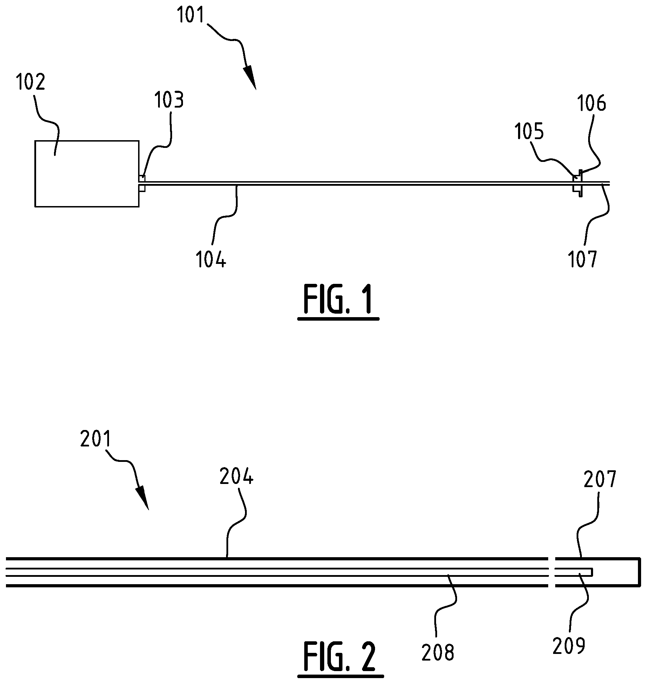

Figure 1 shows a device 101 for regulating the concentration of glucose in the blood of a person. Device 101 comprises a pump 102. Pump 102 comprises at least one container with a substance which affects the concentration of glucose in the blood of the person, for instance glucagon or insulin (not shown). Transport means, in this example in the form of a conduit 104, are connected to pump 102 via a first connector 103, also referred to above as third coupling element. In particular, a first end of conduit 104 is connected to pump 102. At the other end conduit 104 has a second connector 105, also referred to above as first coupling element. Conduit 104 is connected to injecting means, in this example in the form of a cannula 107, by means of the second connector 105 and a third connector 106, also referred to above as second coupling element. The cannula can be connected to the internal part of a person, for instance subcutaneously, so that substance from the container can be injected via conduit 104 and cannula 107 into the body of the person by means of pump 102.

It is noted that the different components of device 101 are shown very simply and/or schematically for the sake of simplicity.

It can for instance be advantageous to flush out conduit 104 with the substance.

This can for instance be advantageous when use is made of a new conduit, optionally with a new cannula 107, so that air can be removed from conduit 104 by flushing out thereof with substance.

In another example a user may find out that the container is empty a few hours after a new infusion set, i.e. conduit 104 and cannula 107, has been taken into use, and exchange it for a filled container. The infusion set is here reused because it has only been in use for a short time. Because the coupling between the container and the conduit has been broken, there may be air in the tube, which air is preferably removed by flushing conduit 104.

Yet another example: it is known that insulin can begin to crystallize after a period of time, which may result in a blockage in the infusion set. This blockage can possibly be removed by flushing out the conduit and/or the conduit can be flushed out in order to ascertain whether the blockage is in the conduit or is in the cannula.

The invention has for its object to make flushing out of the conduit at least safer for the person, regardless of the reason for flushing out the conduit.

Figure 2 shows a part of a device 201 according to a first embodiment. Pump 202 and connectors 203, 205 and 206 have been omitted for the sake of simplicity, but can be embodied as described above with reference to figure 1. In this example conduit 204 comprises an electrically conductive wire 208 which extends through conduit 204 and debouches close to the end of conduit 204 directed toward cannula 207. Cannula 207 comprises a electrically conductive wire 209 which extends through cannula 207 and debouches close to the end of cannula 207 directed toward conduit 204. When conduit 204 and cannula 207 are connected to each other, wires 208 and 209 will come into mutual contact and thus close an electrical circuit. Device 201 can be configured here to detect whether or not an electrical circuit has been closed and therefore whether or not conduit 204 is connected to cannula 207. Device 201, particularly a processor thereof, can use this detection information to control device 201 and particularly the pump 202 thereof in a determined manner. Device 201 can for instance be configured not to flush out conduit 204 with substance when it is detected that conduit 204 is connected to cannula 207, and to flush out conduit 204 with substance when it is detect that conduit 204 is not connected to cannula 207. When conduit 204 is not connected to cannula 207, the device can be connected for medium throughflow to the internal part of the body of the person, making flushing out safe.

It is noted that wires 208 and 209 can alternatively also be light-conducting fibres with which a light circuit can be closed. The device can be embodied in the same way as described with reference to figure 2, wherein the device is configured to detect whether or not the light circuit has been closed and therefore whether or not conduit 204 is connected to cannula 207.

It is further noted that, as alternative to cannula 207 or in addition to cannula 207, pump 202 can be provided with such a wire or fibre. This additional wire or fibre can for instance be used to extend the optional electrical circuit or light circuit to pump 202, wherein the wire or fibre is connected to the other end, not debouching in connector 205, of the wire 208 or fibre. This alternative or additional wire can alternatively or additionally be used to detect whether or not pump 202 and conduit 204 are coupled to each other by ascertaining whether the electrical circuit or light circuit therebetween is closed or not. The optional wire or fibre in pump 202 is not drawn, but the way in which it is arranged will be apparent to the skilled person, i.e. such that the wire or fibre is in contact with the wire 208 or fibre of conduit 204 when conduit 204 is connected for medium throughflow to pump 202. The device can be configured here to detect whether or not an electrical circuit has been closed between the pump and the conduit. The device, particularly a processor thereof, can use this detection information to control the device, and particularly the pump thereof, in a determined manner. In order to prevent leakage, the device can for instance be configured to pump substance only when it is detected that the conduit is connected to the pump. If desired, this can be combined with the detection of whether the conduit is connected to the cannula, so that flushing out can take place when it is detected that the conduit is connected to the pump, but not to the cannula.

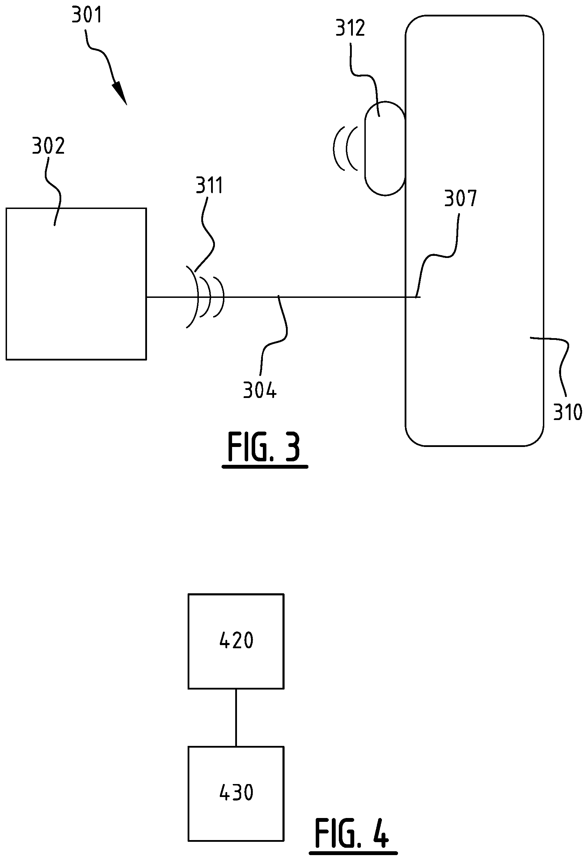

Figure 3 shows a part of a device 201 according to a second embodiment. Connectors 303, 305 and 306 have been omitted for the sake of simplicity, but can be embodied as described above with reference to figure 1 or 2.

Pump 302 is connected via conduit 304 and cannula 307 to the internal part of a body 310 of a person so that pump 302 can inject substance into the body 310. In this example pump 302 is configured to generate a signal 311. For this purpose pump 303 can comprise for instance transmitting means. This signal 311 can be conveyed to a sensor 312 arranged on body 310 via substance in conduit 304 and cannula 307, and via body 310, for instance via a bodily fluid. Sensor 312 can comprise a receiver for receiving this signal. Sensor 312 can further comprise a transmitter for transmitting to pump 302 that the signal generated thereby has been received. When pump 302 generates the signal and sensor 312 receives the signal, it is determined by device 301 that the device 302 is in medium throughflow connection with the internal part of the body 310 of the person. If there is an interruption in the medium throughflow connection anywhere, for instance because conduit 304 is not connected to pump 302 and/or because conduit 304 is not connected to cannula 307 and/or because cannula 307 is not arranged in the body of the person, the signal will not be received by sensor 312, whereby device 301 determines that device 302 is not in medium throughflow connection with the internal part of the body 310 of the person. Device 301, particularly a processor thereof, can use this positive and/or negative detection information to control device 301, and particularly pump 302 thereof, in a determined manner. Positive detection information is understood to mean here that the medium throughflow connection is detected, and negative detection information that the medium throughflow connection is not detected.

Device 301 can for instance be configured not to flush out conduit 304 when the detection information is positive, i.e. there is a medium throughflow connection between device 301 and body 310, and to flush out conduit 304 when the detection information is negative, i.e. there is no medium throughflow connection between device 301 and body 310.

Device 301 can alternatively or additionally be configured to inject the substance into body 310 when the detection information is positive, i.e. there is a medium throughflow connection between device 301 and body 310, and not to pump any substance for injection into the body when the detection information is negative, i.e. there is no medium throughflow connection between device 301 and body 310.

If desired, the device according to the invention can be configured to determine the quantity of substance with which the transport means have to be flushed out in order to flush them out substantially wholly. These means are not shown in the figures, but will be apparent to the skilled person on the basis of the preamble and claims of this text.

Figure 4 shows schematically a method according to an embodiment of the invention in a block diagram. These method steps can be substantially equal to the configuration of the device according to any of the embodiments of the invention.

In step 420 of the method it is detected whether or not a device for regulating the concentration of glucose in the blood of a person, for instance a device according to any of the embodiments of the invention and/or with one or more features as described in this text, is in medium throughflow contact with the internal part of the body of the person via the transport means and the injecting means thereof. In step 430 the device is controlled on the basis of this detection. This control can be configured as desired.

By way of example, the device can be configured not to flush out the transport means with substance in the case of a positive detection, i.e. there is a medium throughflow connection between the device and the body, and/or to flush out the transport means with the substance when flushing out is desired in the case of a negative detection, i.e. there is no medium throughflow connection between the device and the body.

By way of another example, the device can be configured to inject the substance into the body in the case of a positive detection, i.e. there is a medium throughflow connection between the device and the body, and/or not to inject the substance into the body in the case of a negative detection, i.e. there is no medium throughflow connection between the device and the body, in order to thus prevent leakage.

It is noted that the invention is not limited to the shown embodiments but also extends to variants within the scope of the appended claims.

## Claims

1. Device (201, 301) for regulating the concentration of glucose in the blood of a person, comprising:
- pump means (202, 302) for selectively supplying at least one substance which affects the concentration of glucose in the blood of the person;
- injecting means (207, 307) for injecting the substance into the body of the person, such as a cannula or catheter, and
- transport means (204, 304), such as a conduit, tube, hose or the like, which connect the pump means (202, 302) for medium throughflow to the injecting means (207, 307) for the purpose of transporting the substance from the pump means (202, 302) to the injecting means (207, 307);
**characterized in that**
- the device (201, 301) is configured to flush out the transport means (204, 304) with the substance, and
- the device (201, 301) comprises detection means for detecting whether or not the device (201, 301) is in medium throughflow contact with the internal part of the body of the person via the transport means (204, 304) and the injecting means (207, 307), wherein the device (201, 203) is configured to flush out the transport means (204, 304) only when the detection means detect that there is no medium throughflow contact with the internal part of the body of the person at least prior to use of a new set of transport means.

2. Device (201) according to claim 1, wherein the detection means are configured to detect whether or not the transport means (204) are connected for medium throughflow to the injecting means (207), wherein the detection means are configured to determine that there is no contact with the internal part of the body of the person when it is detected that the transport means (204) are not connected for medium throughflow to the injecting means (207).

3. Device (201) according to claim 2, wherein the injecting means (207) and the transport means (204) are each provided with electrical or light-conducting contact means (208, 209), which electrical or light-conducting contact means (208, 209) of the injecting means (207) and the transport means (204) are in mutual contact when the transport means (204) are connected to the injecting means (207) and thus close an electrical circuit or a light circuit, wherein the detection means are configured to determine that there is no contact with the internal part of the body of the person when it is detected that no electrical circuit or light circuit is closed.

4. Device (301) according to claim 1, wherein the device (301) further comprises a sensor (312) arranged on or in the body of the person, wherein one of the pump means (302) and the sensor (312) is configured to generate a signal (311) and wherein the other of the pump means (302) and the sensor (312) is configured to receive a generated signal (311), wherein the detection means are configured to determine that there is no contact with the internal part of the body when a generated signal (311) is not received.

5. Device (201, 301) according to any one of the foregoing claims, wherein the device (201, 301) is configured to determine the quantity of substance with which the transport means (204, 304) have to be flushed out in order to flush them out substantially wholly, and wherein the pump means (202, 302) are configured to pump the determined quantity of substance through the transport means (204, 304).

6. Device (201, 301) according to claim 5, wherein the transport means (204, 304) are provided with indicating means for indicating a length and/or internal cross-sectional dimension and/or internal volume thereof, wherein the device (201, 301) is configured to determine the quantity of substance on the basis of the indicating means.

7. Device (201, 301) according to claim 5 or 6, comprising second detection means for detecting the possible presence of air in the transport means (204, 304), and wherein the pump means (202, 302) are configured to flush out the transport means (204, 304) with the substance until the second detection means detect substantially no air in the transport means (204, 304).

8. Device (201, 301) according to claim 7, wherein the second detection means are configured to convey a signal via substance present in the transport means (204, 304) during use and to receive this signal at an end of the transport means (204, 304) remote from the pump (202, 302), wherein the second detection means are configured to determine that substantially no air is present in the transport means (204, 304) when the signal is received and is therefore conveyed to the end of the transport means (204, 304).

9. Device (201, 301) according to any one of the foregoing claims, wherein the device (201, 301) comprises third detection means for detecting whether or not the transport means (204, 304) are connected to the pump means (201, 301), wherein the pump means (202, 302) are configured to pump substance only when it is detected that the transport means are connected to the pump means (202, 302).

10. Device (201, 301) according to claim 9, wherein the pump means (202, 302) and the transport means (204, 304) are each provided with second electrical or light-conducting contact means, which second electrical or light-conducting contact means of the pump means (202, 302) and the transport means (204, 304) are in mutual contact when the transport means (204, 304) are connected to the pump means (204, 304) and thus close a second electrical circuit or light circuit, wherein the third detection means are configured to detect that the transport means (204, 304) are connected for medium throughflow to the pump means (202, 302) when the second electrical circuit or light circuit is closed.

11. Device (201, 301) according to claim 9 or 10, wherein the device (201, 301) further comprises a second sensor arranged on or in the body of the person, wherein one of the pump means (202, 302) and the second sensor is configured to generate a signal and wherein the other of the pump means (202, 302) and the second sensor is configured to receive a generated signal, wherein the third detection means are configured to determine that the transport means (204, 304) are connected for medium throughflow to the pump means (202, 302) when the generated signal is received.

## Patentansprüche

1. Vorrichtung (201, 301) zum Regulieren der Glucosekonzentration in dem Blut einer Person, umfassend:
- Pumpmittel (202, 302) zum selektiven Zuführen von mindestens einer Substanz, die die Glucosekonzentration in dem Blut der Person beeinflusst;
- Injektionsmittel (207, 307) zum Injizieren der Substanz in den Körper der Person, wie eine Kanüle oder ein Katheter, und
- Transportmittel (204, 304), wie eine Leitung, eine Röhre, ein Schlauch oder dergleichen, die die Pumpmittel (202, 302) für einen Mediumdurchfluss mit den Injektionsmitteln (207, 307) verbinden, zum Zweck eines Transportierens der Substanz von den Pumpmitteln (202, 302) zu den Injektionsmitteln (207, 307);
**dadurch gekennzeichnet, dass**
- die Vorrichtung (201, 301) konfiguriert ist, um die Transportmittel (204, 304) mit der Substanz auszuspülen, und
- die Vorrichtung (201, 301) Erkennungsmittel zum Erkennen umfasst, ob die Vorrichtung (201, 301) über die Transportmittel (204, 304) und die Injektionsmittel (207, 307) in Mediumdurchflusskontakt mit dem inneren Körperteil der Person steht oder nicht, wobei die Vorrichtung (201, 203) konfiguriert ist, um die Transportmittel (204, 304) nur dann auszuspülen, wenn die Erkennungsmittel erkennen, dass kein Mediumdurchflusskontakt mit dem inneren Körperteil der Person, mindestens vor einer Verwendung eines neuen Satzes von Transportmitteln, besteht.

2. Vorrichtung (201) nach Anspruch 1, wobei die Erkennungsmittel konfiguriert sind, um zu erkennen, ob die Transportmittel (204) für einen Mediumdurchfluss mit den Injektionsmitteln (207) verbunden sind oder nicht, wobei die Erkennungsmittel konfiguriert sind, um zu bestimmen, dass kein Kontakt mit dem inneren Körperteil der Person besteht, wenn erkannt wird, dass die Transportmittel (204) nicht für einen Mediumdurchfluss mit den Injektionsmitteln (207) verbunden sind.

3. Vorrichtung (201) nach Anspruch 2, wobei die Injektionsmittel (207) und die Transportmittel (204) jeweils mit elektrischen oder lichtleitenden Kontaktmitteln (208, 209) versehen sind, die elektrischen oder lichtleitenden Kontaktmittel (208, 209) der Injektionsmittel (207) und der Transportmittel (204) in gegenseitigem Kontakt stehen, wenn die Transportmittel (204) mit den Injektionsmitteln (207) verbunden sind, und somit einen Stromkreis oder einen Lichtkreis schließen, wobei die Erkennungsmittel konfiguriert sind, um zu bestimmen, dass kein Kontakt mit dem inneren Körperteil der Person besteht, wenn erkannt wird, dass kein Stromkreis oder Lichtkreis geschlossen ist.

4. Vorrichtung (301) nach Anspruch 1, wobei die Vorrichtung (301) ferner einen Sensor (312) umfasst, der auf oder in dem Körper der Person angeordnet ist, wobei eines der Pumpmittel (302) und der Sensor (312) konfiguriert ist, um ein Signal (311) zu erzeugen, und wobei das andere der Pumpmittel (302) und der Sensor (312) konfiguriert ist, um ein erzeugtes Signal (311) zu empfangen, wobei die Erkennungsmittel konfiguriert sind, um zu bestimmen, dass kein Kontakt mit dem inneren Körperteils besteht, wenn ein erzeugtes Signal (311) nicht empfangen wird.

5. Vorrichtung (201, 301) nach einem der vorstehenden Ansprüche, wobei die Vorrichtung (201, 301) konfiguriert ist, um die Menge an Substanz zu bestimmen, mit der die Transportmittel (204, 304) ausgespült werden müssen, um sie im Wesentlichen vollständig auszuspülen, und wobei die Pumpmittel (202, 302) konfiguriert sind, um die bestimmte Menge an Substanz durch die Transportmittel (204, 304) hindurch zu pumpen.

6. Vorrichtung (201, 301) nach Anspruch 5, wobei die Transportmittel (204, 304) mit Anzeigemitteln zum Anzeigen einer Länge und/oder einer inneren Querschnittsabmessung und/oder eines inneren Volumens davon versehen sind, wobei die Vorrichtung (201, 301) konfiguriert ist, um die Menge an Substanz auf der Grundlage der Anzeigemittel zu bestimmen.

7. Vorrichtung (201, 301) nach Anspruch 5 oder 6, umfassend zweite Erkennungsmittel zum Erkennen der möglichen Anwesenheit von Luft in den Transportmitteln (204, 304), und wobei die Pumpmittel (202, 302) konfiguriert sind, um die Transportmittel (204, 304) mit der Substanz auszuspülen, bis die zweiten Erkennungsmittel im Wesentlichen keine Luft mehr in den Transportmitteln (204, 304) erkennen.

8. Vorrichtung (201, 301) nach Anspruch 7, wobei die zweiten Erkennungsmittel konfiguriert sind, um während einer Verwendung ein Signal über die Substanz zu übertragen, die in den Transportmitteln (204, 304) vorhanden ist, und dieses Signal an einem Ende der Transportmittel (204, 304) zu empfangen, die von der Pumpe (202, 302) entfernt sind, wobei die zweiten Erkennungsmittel konfiguriert sind, um zu bestimmen, dass im Wesentlichen keine Luft in den Transportmitteln (204, 304) vorhanden ist, wenn das Signal empfangen wird, und daher an das Ende der Transportmittel (204, 304) übertragen wird.

9. Vorrichtung (201, 301) nach einem der vorstehenden Ansprüche, wobei die Vorrichtung (201, 301) dritte Erkennungsmittel zum Erkennen umfasst, ob die Transportmittel (204, 304) mit den Pumpmitteln (201, 301) verbunden sind oder nicht, wobei die Pumpmittel (202, 302) konfiguriert sind, um die Substanz nur zu dann zu pumpen, wenn erkannt wird, dass die Transportmittel mit den Pumpmitteln (202, 302) verbunden sind.

10. Vorrichtung (201, 301) nach Anspruch 9, wobei die Pumpmittel (202, 302) und die Transportmittel (204, 304) jeweils mit zweiten elektrischen oder lichtleitenden Kontaktmitteln versehen sind, die zweiten elektrischen oder lichtleitenden Kontaktmittel der Pumpmittel (202, 302) und der Transportmittel (204, 304) in gegenseitigem Kontakt stehen, wenn die Transportmittel (204, 304) mit den Pumpmitteln (204, 304) verbunden sind und somit einen zweiten Stromkreis oder Lichtkreis schließen, wobei die dritten Erkennungsmittel konfiguriert sind, um zu erkennen, dass die Transportmittel (204, 304) für einen Mediumdurchfluss mit den Pumpmitteln (202, 302) verbunden ist, wenn der zweite Stromkreis oder Lichtkreis geschlossen ist.

11. Vorrichtung (201, 301) nach Anspruch 9 oder 10, wobei die Vorrichtung (201, 301) ferner einen zweiten Sensor umfasst, der auf oder in dem Körper der Person angeordnet ist, wobei eines der Pumpmittel (202, 302) und der zweite Sensor konfiguriert ist, um ein Signal zu erzeugen, und wobei das andere der Pumpmittel (202, 302) und der zweite Sensor konfiguriert ist, um ein erzeugtes Signal zu empfangen, wobei die dritten Erkennungsmittel konfiguriert sind, um zu bestimmen, dass die Transportmittel (204, 304) für den Mediumdurchfluss mit den Pumpmitteln (202, 302) verbunden sind, wenn das erzeugte Signal empfangen wird.

## Revendications

1. Dispositif (201, 301) permettant de réguler la concentration de glucose dans le sang d'une personne, comprenant :
- des moyens de pompage (202, 302) pour fournir sélectivement au moins une substance qui affecte la concentration de glucose dans le sang de la personne ;
- des moyens d'injection (207, 307) pour injecter la substance dans le corps de la personne, tels qu'une canule ou un cathéter, et
- des moyens de transport (204, 304), tels qu'un conduit, un tube, un tuyau ou similaire, qui connectent les moyens de pompage (202, 302) pour l'écoulement de fluide aux moyens d'injection (207, 307) afin de transporter la substance des moyens de pompage (202, 302) aux moyens d'injection (207, 307) ;
**caractérisé en ce que**
- le dispositif (201, 301) est configuré pour rincer les moyens de transport (204, 304) avec la substance, et
- le dispositif (201, 301) comprend des moyens de détection pour détecter si le dispositif (201, 301) est en contact d'écoulement de fluide avec la partie interne du corps de la personne par l'intermédiaire des moyens de transport (204, 304) et des moyens d'injection (207, 307), dans lequel le dispositif (201, 203) est configuré pour rincer les moyens de transport (204, 304) uniquement lorsque les moyens de détection détectent qu'il n'y a pas de contact d'écoulement de fluide avec la partie interne du corps de la personne, au moins avant l'utilisation d'un nouvel ensemble de moyens de transport.

2. Dispositif (201) selon la revendication 1, dans lequel les moyens de détection sont configurés pour détecter si les moyens de transport (204) sont connectés pour l'écoulement de fluide aux moyens d'injection (207), dans lequel les moyens de détection sont configurés pour déterminer qu'il n'y a pas de contact avec la partie interne du corps de la personne lorsqu'il est détecté que les moyens de transport (204) ne sont pas connectés pour l'écoulement de fluide aux moyens d'injection (207).

3. Dispositif (201) selon la revendication 2, dans lequel les moyens d'injection (207) et les moyens de transport (204) sont chacun pourvus de moyens de contact électriques ou conducteurs de lumière (208, 209), lesquels moyens de contact électriques ou conducteurs de lumière (208, 209) des moyens d'injection (207) et des moyens de transport (204) sont en contact mutuel lorsque les moyens de transport (204) sont connectés au moyens d'injection (207) et ferment ainsi un circuit électrique ou un circuit lumineux, dans lequel les moyens de détectionsont configurés pour déterminer qu'il n'y a pas de contact avec la partie interne du corps de la personne lorsqu'il est détecté qu'aucun circuit électrique ou lumineux n'est fermé.

4. Dispositif (301) selon la revendication 1, dans lequel le dispositif (301) comprend en outre un capteur (312) agencé sur ou dans le corps de la personne, dans lequel l'un parmi les moyens de pompage (302) et le capteur (312) sont configurés pour générer un signal (311) et dans lequel l'autre parmi les moyens de pompage (302) et le capteur (312) sont configurés pour recevoir un signal généré (311), dans lequel les moyens de détection sont configurés pour déterminer qu'il n'y a pas de contact avec la partie interne du corps lorsqu'un signal généré (311) n'est pas reçu.

5. Dispositif (201, 301) selon l'une quelconque des revendications précédentes, dans lequel le dispositif (201, 301) est configuré pour déterminer la quantité de substance avec laquelle les moyens de transport (204, 304) doivent être rincés afin de les rincer sensiblement entièrement, et dans lequel les moyens de pompage (202, 302) sont configurés pour pomper la quantité déterminée de substance par le biais des moyens de transport (204, 304).

6. Dispositif (201, 301) selon la revendication 5, dans lequel les moyens de transport (204, 304) sont pourvus de moyens d'indication pour indiquer une longueur et/ou une dimension transversale interne et/ou un volume interne associé, dans lequel le dispositif (201, 301) est configuré pour déterminer la quantité de substance sur la base des moyens d'indication.

7. Dispositif (201, 301) selon la revendication 5 ou 6, comprenant des deuxièmes moyens de détection pour détecter la présence éventuelle d'air dans les moyens de transport (204, 304), et dans lequel les moyens de pompage (202, 302) sont configurés pour rincer les moyens de transport (204, 304) avec la substance jusqu'à ce que les deuxièmes moyens de détection ne détectent sensiblement plus d'air dans les moyens de transport (204, 304).

8. Dispositif (201, 301) selon la revendication 7, dans lequel les deuxièmes moyens de détection sont configurés pour transmettre un signal par l'intermédiaire de la substance présente dans les moyens de transport (204, 304) pendant l'utilisation et pour recevoir ce signal au niveau d'une extrémité des moyens de transport (204, 304) éloignée de la pompe (202, 302), dans lequel les deuxièmes moyens de détection sont configurés pour déterminer qu'il n'y a sensiblement pas d'air dans les moyens de transport (204, 304) lorsque le signal est reçu et est donc transmis à l'extrémité des moyens de transport (204, 304).

9. Dispositif (201, 301) selon l'une quelconque des revendications précédentes, dans lequel le dispositif (201, 301) comprend des troisièmes moyens de détection pour détecter si les moyens de transport (204, 304) sont connectés aux moyens de pompage (201, 301), dans lequel les moyens de pompage (202, 302) sont configurés pour pomper la substance uniquement lorsqu'il est détecté que les moyens de transport sont connectés aux moyens de pompage (202, 302).

10. Dispositif (201, 301) selon la revendication 9, dans lequel les moyens de pompage (202, 302) et les moyens de transport (204, 304) sont chacun pourvus de seconds moyens de contact électriques ou conducteurs de lumière, lesquels seconds moyens de contact électriques ou conducteurs de lumière des moyens de pompage (202, 302) et des moyens de transport (204, 304) sont en contact mutuel lorsque les moyens de transport (204, 304) sont connectés aux moyens de pompage (204, 304) et ferment ainsi un second circuit électrique ou lumineux, dans lequel les troisièmes moyens de détection sont configurés pour détecter que les moyens de transport (204, 304) sont connectés aux moyens de pompage (202, 302) pour l'écoulement de fluide lorsque le second circuit électrique ou lumineux est fermé.

11. Dispositif (201, 301) selon la revendication 9 ou 10, dans lequel le dispositif (201, 301) comprend en outre un second capteur agencé sur ou dans le corps de la personne, dans lequel l'un parmi les moyens de pompage (202, 302) et le second capteur sont configurés pour générer un signal et dans lequel l'autre parmi les moyens de pompage (202, 302) et le second capteur sont configurés pour recevoir un signal généré, dans lequel les troisièmes moyens de détection sont configurés pour déterminer que les moyens de transport (204, 304) sont connectés pour l'écoulement de fluide vers les moyens de pompage (202, 302) lorsque le signal généré est reçu.
